# EUROPEAN PATENT APPLICATION

(11) **EP 2 082 760 A1**
(43) Date of publication of application: **29.07.2009**
(21) Application number: 08001294.1
(22) Date of filing: 24.01.2008
(51) Int. Cl.: A61M 15/00

(54) **Inhaler**

(71) Applicant: BOEHRINGER INGELHEIM INTERNATIONAL GMBH, 55216 Ingelheim (DE); Vectura Delivery Devices Limited, Chippenham Wiltshire SN14 6FH (GB)
(72) Inventor: Wachtel, Herbert, 55216 Ingelheim am Rhein (DE); Thoemmes, Ralf, 55216 Ingelheim am Rhein (DE); Frentzel-Beyme, Jessica, 55216 Ingelheim am Rhein (DE); Besseler, Jens, 55216 Ingelheim am Rhein (DE); Staniforth, John Nicholas, Claverton, Bath, BA2 7BG (GB)
(74) Representative: Sharrock, Daniel John

(57) **Abstract**

A passive inhaler (1) for delivery of a powder-form inhalation formulation from a blister strip (2) with a plurality of blister pockets (3) is proposed. The inhaler is adapted to move the blister strip onward by two blister pockets in each indexing operation or to receive two blister strips. The inhaler comprises two piercing elements (17a,17b) for simultaneously puncturing two blister pockets, wherein the doses of the punctured blister pockets can be delivered simultaneously via a common mouthpiece (8).

## Description

The present invention relates to an inhaler according to the preamble of claim 1, 2 or 8.

The present invention relates to an inhaler for delivery of a powder-form inhalation formulation from a blister strip with a plurality of blister pockets (also called blisters) containing the inhalation formulation in doses.

GB 2 407 042 A discloses an inhaler with a rolled-up blister strip. The inhaler comprises a manually operated, pivotable actuator, which operates a conveyor for stepwise moving the blister strip. The actuator supports a piercer and an associated mouthpiece. By pivoting the actuator, the blister strip can be moved forward and blister pockets of the blister strip can be pierced one after the other. When a patient breathes in, an air stream passes through the previously pierced blister pocket, with the result that the inhalation formulation in the blister pocket mixes with the air and is discharged to the patient.

The present invention relates to passive inhalers, i.e. inhalers where the patient or user breathes in to generate an air stream, which entrains the inhalation formulation and forms the desired aerosol.

Object of the present invention is to provide an inhaler which can deliver different inhalation formulations or double-doses during each use.

The above object is achieved by an inhaler according to claim 1, 2 or 8. Advantageous embodiments are subject of the subclaims.

According to one aspect of the present invention, the inhaler and/or conveyor are/is adapted to move the blister strip onward by two blister pockets in each step or indexing operation. Thus, two different inhalation formulations from two adjacent blister pockets can be delivered (discharged) during each use of the inhaler. Alternatively, two doses of the same formulation can be discharged simultaneously.

According to a further aspect of the present invention, the inhaler and/or conveyer are adapted for simultaneous receipt of two blister strips so that a dose of inhalation formulations of each blister strip can be discharged simultaneously. Thus, it is possible to discharge or deliver two different inhalation formulations, namely each from one blister pocket of one of the two blister strips. Alternatively, two doses of the same inhalation formulation can be discharged simultaneously from the two blister strips.

According to another aspect of the present invention, the inhaler comprises two piercing elements for puncturing the lids of the two blister pockets or compartments of one blister pocket simultaneously and/or for the same delivery, preferably wherein the doses of the two punctured blister pockets or compartments can be delivered simultaneously via a mouthpiece. This allows simultaneous delivery of two different inhalation formulations or components or of two doses of the same formulation with very simple operation.

According to the present invention, the different inhalation formulations can be stored separately, namely in different blister strips, blister pockets or compartments. Thus, even incompatible inhalation formulations or components can be used. Only during inhalation, the different inhalation formulations or components are mixed. In particular, the different inhalation formulations or components allow a combination or synergistic effect and/or the administration of a combination medicament or the combination of different formulations or medicaments.

Further aspects, features, properties and advantages of the present invention are described in the claims and the subsequent description of preferred embodiments, with reference to the drawing. There are shown in:
- Fig. 1: a schematic sectional view of an inhaler without mouthpiece cover for explanatory purposes;
- Fig. 2: a schematic sectional representation of the inhaler with closed mouthpiece cover for explanatory purposes;

- Fig. 3: an enlarged sectional view of the inhaler according to the present invention in the region of a mouthpiece and a piercing member;
- Fig. 4: an enlarged sectional view of another inhaler according to the present invention in the region of the mouthpiece and a piercing member similar to fig, 3;
- Fig. 5: a schematic perspective view of a further inhaler according to the present invention; and
- Fig. 6: an enlarged sectional view of a piercing member and associated blister strips of the further inhaler according to fig. 5.

In the Figures, the same reference numbers are used for identical or similar parts, even if a repeated description is omitted. In particular identical or corresponding advantages and properties then also result or may be achieved.

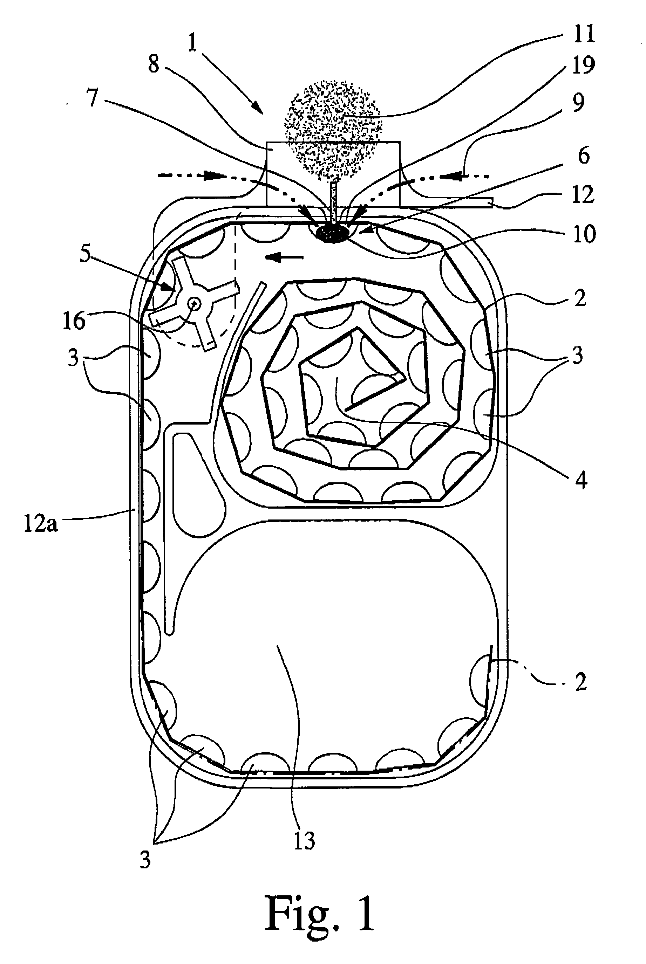

Fig. 1 shows in a schematic sectional representation an inhaler 1. Preferably, the inhaler 1 is portable, works only mechanically and/or is hand-held.

The inhaler 1 serves to deliver a powdered inhalation formulation from a band-shaped blister strip 2. The blister strip 2 is finite, not forming an endless or closed loop. It has a large number of blister pockets 3 respectively containing directly a dose of the loose inhalation formulation. Thus, the formulation is pre-metered.

The inhaler 1 has a reservoir 4 for the still unused blister strip 2 with closed (sealed) blister pockets 3. The blister strip 3 is rolled up or wound up in the reservoir 4. In the representation example the reservoir 4 is formed such that the blister strip 2 can be moved outwards or pulled out of the reservoir 4 as easily as possible.

In the representation example the blister strip 2 is directly received in the reservoir 4. However, instead of this a cassette, a container, a drum or suchlike can also be fitted or inserted with the blister strip 2 into the inhaler 1 or the reservoir 4.

The inhaler 1 has a conveyor 5 for stepwise onward movement of the blister strip 2 in direction of arrow 5a by one blister pocket 3 in each case, in order to feed the blister pockets 3 successively to an opening and/or removal position 6 where the respective blister pocket 3 is opened and can be emptied.

The blister pockets 3 can be opened respectively preferably by means of a piercing member 7 which punctures or cuts open a lid 19 of the respectively aligned blister pocket 3 in position 6. The piercing member 7 fluidically connects the respective blister pocket 3 with an adjacent mouthpiece 8 of the inhaler 1.

During or for inhalation a patient or user, not represented, places the mouthpiece 8 in his mouth and breathes in. The respectively opened blister pocket 3, into which the piercing member 7 extends, is thereby emptied by sucking in. An air stream 9 of ambient air is sucked in and passed through the opened blister pocket 3 such that the loose powder 10 (forming the inhalation formulation and being schematically shown in Fig. 1 only in the actually opened blister pocket 3 below mouthpiece 8) is dispensed with the sucked-in ambient air as an aerosol cloud 11 via the mouthpiece 8. This situation is schematically represented in Fig. 1.

The inhaler 1 has a preferably manually actuatable, lever-like actuator 12 being pivotally mounted to a housing 12a of the inhaler 1. The piercing member 7 and the mouthpiece 8 are attached to and supported by the actuator 12.

The actuator 12 is operable (pivotable) to cause the piercing member 7 to puncture the lid 19 of the respectively aligned blister pocket 3 in position 6 below the mouthpiece 8.

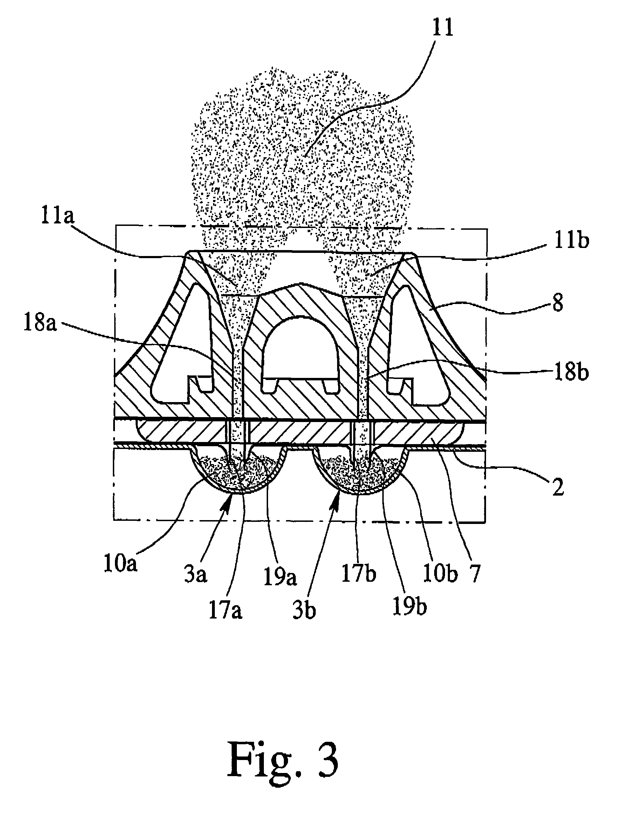

When the actuator 12 swivels from the position shown in Fig. 1 (here anticlockwise) to the partially opened position shown in Fig. 3, the piercing member 7 is withdrawn from the last-pierced blister pocket 3.

Then, the blister strip 2 is moved forward by one blister pocket 3, so that the next blister pocket 3 is moved in position 6. This will be explained in more detail later.

When the actuator 12 swivels back into the position shown in Fig. 1, i.e. is manually moved back, the next aligned blister pocket 3 of the blister strip 2 is punctured by the piercing member 7 and thereby opened. Then, the inhaler 1 is activated and the next inhalation can take place.

The inhaler 1 has a receiving space or apparatus 13 to receive or store the used part of the blister strip 2. The receiving space or apparatus 13 is formed such that the used part can be wound up. Fig. 1 shows a situation with essentially filled reservoir 4 and still essentially empty receiving space 13.

The conveyor 5 comprises a conveying wheel 14, which can engage between the blister pockets 3 and thus convey the blister strip 2 in form-locking or form-fit manner. This allows very secure or precise moving or indexing of the blister strip 2 as desired and/or necessary.

The conveyor 5 or its conveying wheel 14 is arranged between the reservoir 4 and the receiving apparatus 13, in particular between the removal position 6 and the receiving apparatus 13, thus after the emptying of the blister pockets 3.

The pivot axis of the actuator or lever 12 is coaxial with the rotation axis of the conveying wheel 14. In particular, the actuator or lever 12 may be supported by an axle of the conveying wheel 14.

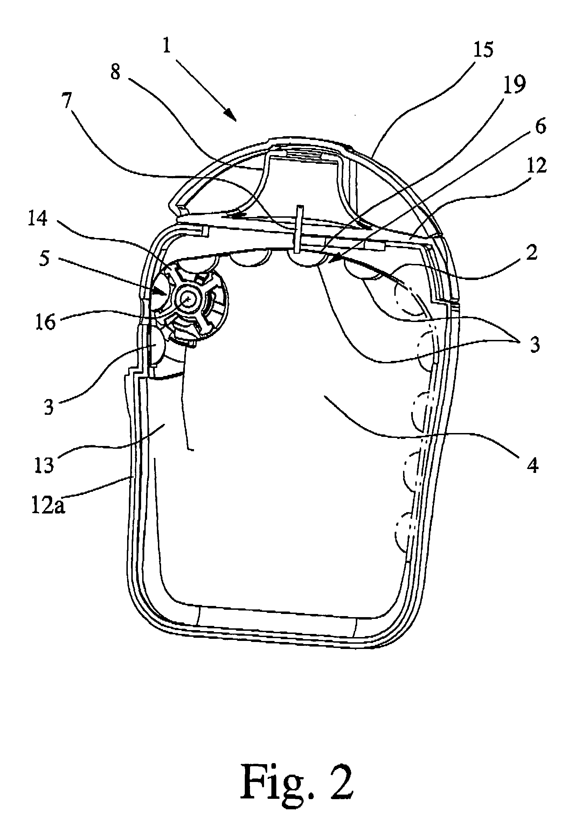

The inhaler 1 comprises a mouthpiece cover 15. The mouthpiece cover 15 is not shown in Fig. 1, which explains only the basic principle of the inhaler 1, but in Fig. 2, which shows a more realistic, but still schematic sectional view of the inhaler 1. Fig. 2 shows the inhaler 1 with closed mouthpiece cover 15, wherein the blister strip 2 has been partly omitted for illustration purposes.
Fig. 3 shows the inhaler 1 with completely opened mouthpiece cover 15.

The mouthpiece cover 15 is pivotable around a cover axis 16, which is indicated in Fig. 2 and 3 and extends perpendicular to the drawing plane in the present representation.

The pivot axis of the actuator 12 extends coaxial to or with the cover axis 16. The rotation axis of the conveying wheel 14 extends coaxial to the cover axis 16 and to pivot axis of the actuator 12.

The conveyor 5 or its conveying wheel 14 is driven by the mouthpiece cover 15, namely by the pivotal movement of the mouthpiece cover 15. In particular, the blister strip 2 is moved forward, when the mouthpiece cover 15 is opened. Preferably, only part of the opening movement of the mouthpiece cover 15 actuates or operates the conveyor 5 or its conveying wheel 14 to move the blister strip 2 forward.

When the mouthpiece cover 15 is opened starting with the completely closed position shown in Fig. 2, in a first phase of the opening movement, for example up to a first angle of about 10, 20, 30 or 40 degrees, the blister strip 2 is not moved due to a respective freewheel (not shown) between the mouthpiece cover 15 and the conveying wheel 14.

First of all, the actuator 12 has to be moved or opened in order to withdraw the piercing member 7 from the previously pierced and usually/already emptied blister pocket 3. This opening movement of the actuator can be performed manually. However, the actuator 12 preferably opens automatically or together with the mouthpiece cover 15 when opening the mouthpiece cover 15.

In particular, the mouthpiece cover 15 is opened up to the first angle. When the mouthpiece cover 15 reaches or exceeds the opened position of the actuator 12, the actuator 12 can flip automatically open into its opened position shown in Fig. 2, in particular due to a biasing or spring means (not shown) or the like. However, it also possible and preferred that the actuator 12 moves jointly with the mouthpiece cover 15 in the first phase of the opening movement (e.g. due to a ratchet mechanism, a spring, a coupling means or the like) until the actuator 12 reaches its opened position or the first angle.

The opened position of the actuator 12 is preferably set such that the piercing member 7 is not exposed to the exterior and/or that the inhaler 1 is not completely opened in order to avoid or at least minimize any potential external influences.

In order to limit the open position of the actuator 12, the opening or pivot range of the actuator 12 is smaller than the one of the mouthpiece cover 15 and/or is restricted to preferably at most 20 degrees, in particular to about 10 degrees or less.

However, it is also possible that the actuator 12 is not limited in its opening position, but can open or pivot as far as the mouthpiece cover 15, in particular jointly with the mouthpiece cover 15.

During the further opening (second phase) of the mouthpiece cover 15, the conveyor 5 or its conveying wheel 14 is actuated to move or index the blister strip 2 onward to the next blister pocket(s) 3 that shall be emptied. This blister movement happens preferably up to the complete opening of the mouthpiece cover 15.

When the mouthpiece cover 15 has been fully opened and the next blister pocket 3 has been moved in position 6, the actuator 12 can be pivoted back, i.e. closed, in order to pierce the already aligned, still closed blister pocket 3. Then, the inhaler 1 is ready for inhalation, i.e. activated as already described.

After inhalation, the inhaler 1 can be closed by pivoting back the mouthpiece cover 15 into its closed position.

In order to operate the conveyor 5 or its conveying wheel 14 by movement of the mouthpiece cover 15 as described above or in any other suitable manner, the mouthpiece cover 15 is coupled with the conveyor 5, in particular the conveying wheel 14, via the already mentioned freewheel and a suitable transmission, a slipping clutch or any other suitable coupling or the like.

Preferably, the freewheel, transmission, coupling or the like is integrated into or located adjacent to the conveying wheel 14 or a respective axle.

Fig. 3 shows an enlarged sectional view of the inhaler 1 according to the present invention in the region of the piercing member 7 and the mouthpiece 8.

The inhaler 1 - here its piercing member 7 - comprises two piercing elements 17a, 17b for preferably simultaneously puncturing lids 19a, 19b of two blister pockets 3a, 3b as schematically shown (not in scale) in Fig. 3.

Preferably, the inhaler 1 comprises two feeding paths 18a, 18b fluidically connectable to the two opened blister pockets 3a, 3b or fluidically connected to the two piercing elements 17a, 17b.

The two punctured blister pockets 3a, 3b may comprise the same inhalation formulation 10 or different inhalation formulations 10a and 10b. In the latter case, the blister strip 2 comprises preferably alternatively the different inhalation formulations 10a and 10b in its blister pockets 3.

Preferably, the doses of the two simultaneously punctured blister pockets 3 can be delivered simultaneously via the mouthpiece 8 as schematically shown in Fig. 3. In particular, the inhaler 1 is designed such that by breathing in during inhalation, ambient air can be simultaneously sucked in through the two opened blister pockets 3a, 3b and through the two feeding paths 18a and 18b into the mouthpiece 8.

Preferably, each inhalation formulation - here powder 10a and 10b - is dispensed during inhalation as a respective aerosol cloud 11a and 11b, wherein the aerosol clouds 11a and 11b mix and form an common aerosol cloud 11 preferably within the common mouthpiece 8 as schematically shown in Fig. 3 or thereafter.

In the preferred embodiment, the piercing member 7 comprises or forms both piercing elements 17a, 17b. However, the piercing element 17a, 17b could also be formed by separate parts, by the mouthpiece 8 or any other component of the inhaler 1.

The feeding paths 18a, 18b are preferably formed by respective channels, ducts or the like. Preferably, the feeding paths 18a, 18b are formed in or by the mouthpiece 8. In particular, the feeding paths 18a and 18b end within the mouthpiece 8, preferably such that the mixing zone is formed within the inhaler 1 or mouthpiece 8 and/or adjacent to the outlet of the mouthpiece 8.

Preferably, the inhaler 1 or its conveyor 5 is designed or adapted to move the blister strip 2 onward always by two blister pockets 3a, 3b in each step or indexing operation. Thus, respectively two unused (closed) blister pockets 3a, 3b are aligned or moved under the piercing elements 17a, 17b in each indexing step or operation.

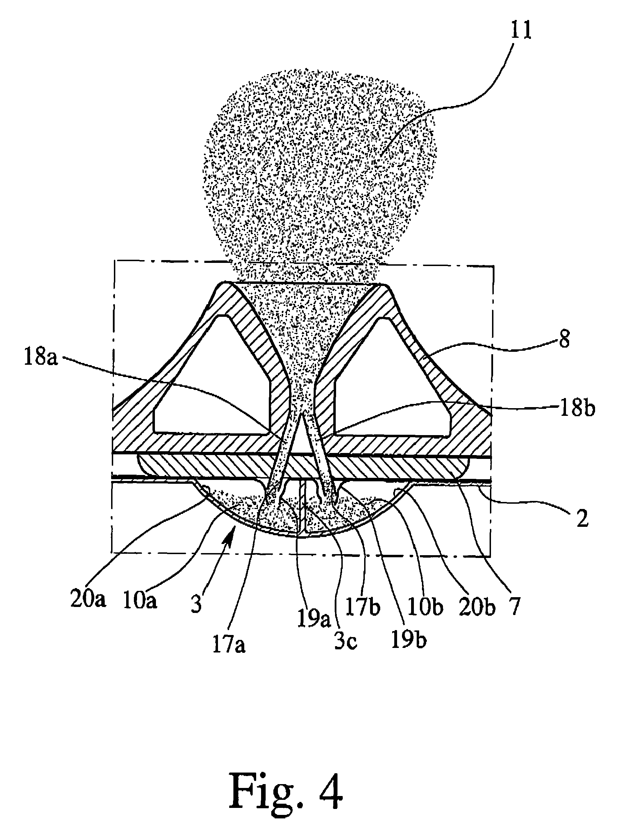

Fig. 4 shows in a schematic sectional view similar to Fig. 3 another embodiment of the inhaler 1 according to the present invention. Here, each blister pocket 3 comprises or forms two separate compartments 20a and 20b. In particular, the compartments 20a and 20b are formed by a partition wall 3c dividing the blister pocket 3 into the compartments 20a and 20b. However, other constructional solutions are possible as well.

Both compartments 20a and 20b of one blister pocket 3 may be covered by one common lid 19. However, respective portions 19a and 19b of the lid 19, or with other words individual lids 19a and 19b, are pierced preferably by individual and separate piercing elements 17a and 17b similar to the embodiment according to Fig. 3.

The compartments 20a and 20b may contain the same inhalation formulation or, preferably, different inhalation formulations (powders 10a and 10b).

The embodiment according to Fig. 4 allows a very compact construction, in particular in comparison to the embodiment according to Fig. 3. Further, it is sufficient to move the blister strip 2 onward only in steps of one blister pocket 3 and not in steps of two blister pockets 3 as it is the case in the embodiment according to Fig. 3.

The two compartments 20a and 20b may be aligned or arranged one after the other in the longitudinal direction of the blister strip 2 or in the direction of onward movement 5a. However, it is also possible to arrange the associated compartments 20a and 20b transversally, in particular perpendicular, to this direction.

The explanations and possibilities mentioned with regard to the embodiment according to Fig. 3 preferably apply in a similar manner to the embodiment according to Fig. 4 as well, as to the further embodiment described below.

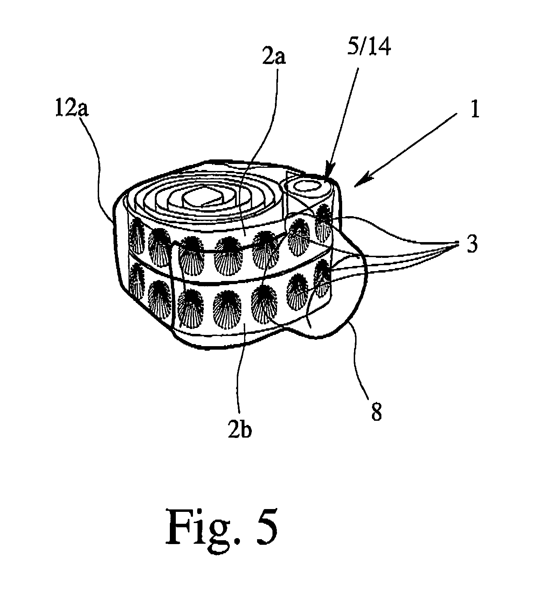

Fig. 5 shows in a schematic perspective view a further embodiment of the inhaler 1 according to the present invention. The inhaler 1 comprises or is adapted to receive two blister strips 2a and 2b.

The blister strips 2a, 2b are placed preferably side-by-side and/or preferably extend parallel to each other.

Preferably, the blister strips 2a and 2b are coiled, in particular wound in the same sense, as schematically shown in fig. 5.

Preferably, the blister strips 2a and 2b are coiled coaxially. Preferably, the coils of the blister strips 2a and 2b are arranged one above the other and/or are of essentially the same size.

The two blister strips 2a and 2b enable the simultaneous delivery of two doses of inhalation formulations stored in separate blister pockets 3 of the separate blister strips 2a and 2b.

Preferably, these formulations would be two different formulations such that a beneficial combination of drugs can be delivered. A particular aspect of the invention is that the different formulations can be filled and sealed into the two separate blister strips 2a and 2b independently. After filling and sealing, the two blister strips 2a and 2b are brought together in the assembly of the inhaler 1. This avoids combining the filling of the two different compounds into one device, inhaler 1 or blister strip 2.

The blister strips 2a and 2b are preferably conveyed or moved onward by a common conveyer 5, in particular a common conveying wheel 14. In particular, the conveying wheel 14 is dimensioned in axial directions such that both blister strips 2a and 2b can be driven simultaneously. However, other constructional solutions are possible as well.

Preferably, the inhaler 1 comprises a common receiving apparatus 13 for receiving the two blister strips 2a and 2b. However, different receiving apparatuses 13 could be used alternatively.

Preferably, the blister strips 2a and 2b are moved parallel to each other and/or in two separate or different planes. This applies preferably for the stored unused blister coils or strips 2a and 2b and/or for the used blister coils or strips 2a and 2b.

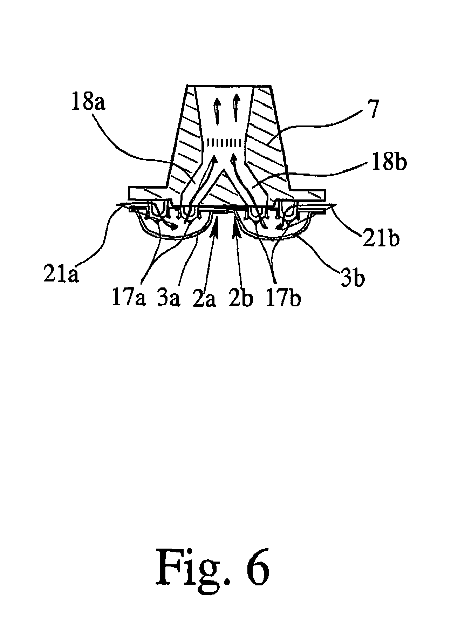

During use, the dose of the inhalation formulation from each blister strip 2a and 2b can be inhaled simultaneously by a user. In particular, the mouthpiece cover 15 or actuator 12 is operable to move simultaneously both blister strips 2a and 2b into alignment with the piercing member 7 (not shown in fig. 5). Further, the actuator 12 is operable to cause the piercing member 7, which comprises piercing elements 17a and 17b to puncture simultaneously the lids of respective blister pockets 3a and 3b of the blisters 2a and 2b as schematically shown in fig. 6. When a user inhales through the mouthpiece 8, an airflow through each opened blister pocket 3a and 3b is generated which entrains the doses contained therein, carries them out of the blister pockets 3a and 3b, and delivers them via the mouthpiece 8 into the user's airway.

In general, the used blister pockets 3a and 3b may either be removed from the inhaler 1 or retained within the housing 12a.

Fig. 6 shows the piercing member 7, which can directly form the mouthpiece 8 or can be connected to or inserted into the mouthpiece 8.

This piercing member 7 is a little bit different in design or construction than the piercing member of fig. 3 and 4. In order to avoid repetition of the description, reference is made to the above description relating to the piercing member 7 shown in fig. 3 and 4.

Fig. 6 shows in addition the flow path or air stream 21 a and 21b that is sucked respectively into the opened blister pockets 3a and 3b, respectively. The piercing member 7 or piercing elements 17a and 17b comprise respective means to puncture or open the lids of the blister pockets 3a and 3b so that suitable inlet openings for the air streams 21 a and 21b are formed and that suitable outlet openings are formed through which the air can stream together with the entrained formulations out of the blister pockets 3a and 3b through the feeding paths 18a and 18b and into the subsequent, preferably common outlet area or mouthpiece 8. However, the air streams and respective formulations can also be guided separately to the end of the mouthpiece 8, if preferred or necessary.

It has to be noted that instead of the two blister strips 2a and 2b, also a single blister strip 2 could be used wherein the blister pockets 3 may be arranged in two parallel rows or lines along the length of the blister strip 2. The rows or lines of blister pockets 3 may contain different inhalation formulations or components or the same inhalation formulation as mentioned above.

In general, two different inhalation formulations may be delivered simultaneously as already mentioned. For example, a long acting beta agonist (LABA) and an inhaled corticosteroid (ICS) may be delivered together. This can potentially provide improved treatment and reduce the number of inhalers that a patient needs to carry around.

One blister strip may even contain a combination formulation of a LABA and an ICS, with the other strip containing a different single or combination formulation, thus offering triple or quadruple therapy options. This applies also in case of the single blister strip 2 described above.

As already mentioned, the two blister strips 2a and 2b could also carry the same inhalation formulation. In this case, the dose capacity of the inhaler 1 could be increased.

Individual features and aspects of the described embodiments and alternatives may be combined as desired.

Preferably, the terms "blister strip" and "blister pockets" have to be understood in a very broad sense to cover also other kinds of storage means with receptacles or even bulk storages for the formulation.

### List of reference numbers

- 1: inhaler
- 2: blister strip
- 2a: blister strip
- 2b: blister strip
- 3: blister pocket
- 3a: blister pocket
- 3b: blister pocket
- 3c: partition wall
- 4: reservoir
- 5: conveyor
- 5a: onward movement
- 6: opening and/or removal position
- 7: piercing member
- 8: mouthpiece
- 9: air stream
- 10: powder
- 10a: powder
- 10b: powder
- 11: aerosol cloud
- 11a: aerosol cloud
- 11b: aerosol cloud
- 12: actuator
- 12a: housing
- 13: receiving apparatus
- 14: conveying wheel
- 15: mouthpiece cover
- 16: cover axis
- 17a: piercing element
- 17b: piercing element
- 18a: feeding path
- 18b: feeding path
- 19: lid
- 19a: lid
- 19b: lid
- 20a: compartment
- 20b: compartment
- 21a: air stream
- 21b: air stream

## Claims

1. Inhaler (1) for delivery of an inhalation formulation from a preferably band-shaped blister strip (2) with a plurality of blister pockets (3) containing the inhalation formulation in doses, comprising:
a conveyor (5) for stepwise onward movement of the blister strip (2),
preferably a piercing member (7) to puncture a lid (19) of an aligned blister pocket (3), and
preferably a mouthpiece (8),
the inhaler (1) being designed such that - preferably by breathing in during inhalation - an air stream (9) of ambient air can be sucked or delivered in order to discharge the respective dose from an opened blister pocket (3) and to deliver it with the ambient air as an aerosol cloud (11),
**characterized in**
**that** the inhaler (1) and/or conveyor (5) are adapted to move the blister strip (2) onward by two blister pockets (3a, 3b) in each step or indexing operation.

2. Inhaler (1) for delivery of an inhalation formulation from a preferably band-shaped blister strip (2) with a plurality of blister pockets (3) containing the inhalation formulation in doses, comprising:
preferably a conveyor (5) for stepwise onward movement of the blister strip (2),
preferably a piercing member (7) to puncture a lid (19) of an aligned blister pocket (3), and
an endpiece, preferably a mouthpiece (8),
the inhaler (1) being designed such that - preferably by breathing in during inhalation - an air stream (9) of ambient air can be sucked or delivered in order to discharge the respective dose from an opened blister pocket (3) and to deliver it with the ambient air as an aerosol cloud (11),
**characterized in**
**that** the inhaler (1) and/or conveyor (5) are adapted for simultaneous receipt of two blister strips (2) so that a dose of inhalation formulation of each blister strip (2) can be discharged simultaneously via the endpiece or mouthpiece (8).

3. Inhaler according to claim 2, **characterized in that** the two blister strips (2) are placed side-by-side and/or extend parallel to each other.

4. Inhaler according to claim 2 or 3, **characterized in that** the two blister strips (2) are coiled and preferably wound in the same sense and/or are coiled coaxially.

5. Inhaler according to one of claims 2 to 4, **characterized in that** the conveying wheel (14) is adapted to commonly convey the two blister strips (2).

6. Inhaler according to one of claims 2 to 5, **characterized in that** the inhaler (1) comprises a common receiving apparatus (13) for receiving the two blister strips (2).

7. Inhaler according to one of the preceding claims, **characterized in that** the inhaler (1) comprises two piercing elements (17a, 17b) for puncturing the lids (19a, 19b) of two blister pockets (3a, 3b) simultaneously, wherein the doses of the two punctured blister pockets (3a, 3b) can be delivered simultaneously via the mouthpiece (8).

8. Inhaler (1) for delivery of an inhalation formulation from a preferably band-shaped blister strip (2) with a plurality of blister pockets (3) containing the inhalation formulation in doses, comprising:
preferably a conveyor (5) for stepwise onward movement of the blister strip (2),
a piercing member (7) to puncture a lid (19) of an aligned blister pocket (3), and
preferably a mouthpiece (8),
the inhaler (1) being designed such that - preferably by breathing in during inhalation - an air stream (9) of ambient air can be sucked or delivered in order to discharge the respective dose from an opened blister pocket (3) and to deliver it with the ambient air as an aerosol cloud (11),
**characterized in**
**that** the inhaler (1) comprises two piercing elements (17a, 17b) for puncturing the lids (19a, 19b) of two blister pockets (3a, 3b) or of two compartments (20a, 20b) of one blister pocket (3) simultaneously and/or for the same delivery, in particular wherein the doses of the two punctured blister pockets (3a, 3b) or compartments (20a, 20b) can be delivered simultaneously preferably via the mouthpiece (8).

9. Inhaler according to claim 7 or 8, **characterized in that** the piercing member (7) comprises or forms both piercing elements (17a, 17b).

10. Inhaler according to one of claims 7 to 9, **characterized in that** the inhaler (1) comprises two feeding paths (18a, 18b) fluidically connectable to the two opened blister pockets (3a, 3b) or compartments (20a, 20b) or connected to the two piercing elements (17a, 17b).

11. Inhaler according to claim 10, **characterized in that** the inhaler (1) is designed such that by breathing in during inhalation ambient air can be simultaneously sucked in through the two opened blister pockets (3a, 3b) or compartments (20a, 20b) and through the feeding paths (18a, 18b) into the mouthpiece (8).

12. Inhaler according to claim 10 or 11, **characterized in that** piercing member (7) and/or the mouthpiece (8) form the two feeding paths (18a, 18b).

13. Inhaler according to one of claims 10 to 12, **characterized in that** the two feeding paths (18a, 18b) end in the mouthpiece (8).
